Europäisches Patentamt

European Patent Office

Office européen des brevets

(19)

(11) Veröffentlichungsnummer: **0 212 084**
A1

# EUROPÄISCHE PATENTANMELDUNG

(21) Anmeldenummer: **86107054.8**

(22) Anmeldetag: **23.05.86**

(51) Int. Cl.⁴: **A 61 F 2/30**

(30) Priorität: **15.07.85 CH 3064/85**

(43) Veröffentlichungstag der Anmeldung: **04.03.87**
Patentblatt 87/10

(84) Benannte Vertragsstaaten: **AT DE FR GB IT**

(71) Anmelder: **GEBRÜDER SULZER
AKTIENGESELLSCHAFT, Zürcherstrasse 9,
CH-8401 Winterthur (CH)**
Anmelder: **Protek AG, Stadtbachstrasse 64,
CH-3001 Bern (CH)**

(72) Erfinder: **Spotorno, Lorenzo, Dr.-med., Ospedale Riuniti,
I-17024 Finale Ligure (IT)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

(54) **Gerader, allseitig abgerundeter Schaft zur Verankerung einer Hüftgelenksprothese mit Hilfe von Knochenzement.**

(57) Der allseitig abgerundete Schaft (1) ist in seinem proximalen Bereich mit Vertiefungen (5) als Verankerungsstruktur versehen. Die Mantelflächen (6) der Vertiefungen (5) stehen senkrecht auf der Schaftoberfläche.

Bei Belastungen in Richtung der Schaftachse, d.h. in der Richtung der Hauptbelastungen (8) verbleibt auch nach Schwinden des Knochenzements (7) eine stufenförmige Auflage zwischen Vertiefung (5) und Knochenzement (7). Diese Auflage steht senkrecht auf der Hauptbelastungsrichtung (8). Es treten daher keine Belastungskomponenten auf, die den Knochenzement (7) aus der Vertiefung (5) hinausdrängen.

ACTORUM AG

P. 5970/Wg/IS

Gebrüder Sulzer, Aktiengesellschaft, Winterthur/Schweiz
Protek AG, Stadtbachstr. 64, 3001 Bern

Gerader, allseitiger abgerundeter Schaft zur Verankerung
einer Hüftgelenksprothese mit Hilfe von Knochenzement

Die Erfindung betrifft einen geraden, allseitig abgerundeten
Schaft zur Verankerung einer Gelenkendoprothese mit Hilfe
von Knochenzement, welcher Schaft auf der Oberfläche mit
einer Verankerungsstruktur aus einzelnen Vertiefungen versehen ist.

Ein Schaft wie er vorstehend beschrieben ist, ist beispielsweise für eine Hüftgelenksprothese bekannt aus der DE-PS
837 294. Die dort beschriebenen Oberflächenstrukturen bestehen unter anderem aus Längsrillen, die parallel zur
Schaftachse verlaufen oder aus einzelnen Vertiefungen, die
die Form von hohlen Kugelkalotten haben. Werden Schäfte mit
einer derartigen Struktur mit Hilfe eines Knochenzementbettes
verankert, so besteht die Gefahr von Schaftlockerungen; denn
der Knochenzement ist bekanntlich einer Schwindung unterworfen, so dass er sich teilweise aus den Vertiefungen der
Struktur "zurückzieht". Gegen die - bei einem geraden Schaft
vor allem in Richtung der Längsachse wirkenden - Belastungskräfte sind die Längsrillen praktisch wirkungslos; sie behindern nur unerwünschte Rotationen. Aus kugelförmigen
singulären Vertiefungen wird der geschwundene Zement durch
die Belastungskräfte in Richtung der Schaftachse - ebenso
wie aus anderen Vertiefungen mit zur Belastungsrichtung schrägen Wänden - nach aussen herausgedrückt, wodurch die bei der Implantation in den Vertiefungen des Schaftes gebildeten "Vorsprünge" des Knochenzementes leicht aus diesen Vertiefungen herausrutschen. Dies führt, besonders wegen der dauernden Wechsel zwischen Be- und Entlastungen, schliesslich zu Schaftlockerungen.

Aufgabe der Erfindung ist es daher, einen Schaft der eingangs beschriebenen Art mit einer Oberflächenstruktur zu versehen, von der die Belastungen in Richtung der Längsachse auf den Knochenzement trotz seines Schwindens weitergeleitet werden, ohne dass der Zement dabei aus der Struktur herausgedrückt wird. Diese Aufgabe wird mit der Erfindung dadurch gelöst, die Vertiefungen, die mindestens im proximalen Bereich des Schaftes angeordnet sind, mit ihren Mantelflächen mindestens nahezu senkrecht zur Oberfläche des Schaftes verlaufen.

Bei einer derartigen Ausgestaltung der Vertiefungen bildet jede Vertiefung einen im wesentlichen senkrecht zur Belastungs- richtung stehenden stufenförmigen Absatz, auf dem sich auch der geschwundene Knochenzement abstützt, ohne dass ihn dabei eine nach aussen gerichtete Kraftkomponente aus der Vertiefung herausdrückt.

Wie aus Versuchen ermittelt worden ist, hat es sich als vorteilhaft erwiesen, wenn die Tiefe der Vertiefungen, also die "Stufenhöhe", absolut mindestens 1 mm beträgt, während ihre Durchmesser zweckmässigerweise das 2- bis 4-fache ihrer Tiefe betragen können.

Im folgenden wird die Erfindung anhand einer Hüftgelenksprothese als Ausführungsbeispiel im Zusammenhang mit der Zeichnung näher erläutert.

Fig. 1 ist eine Ansicht von anterior oder posterior
auf einen mit der neuen Struktur versehenen
Schaft für eine Hüftgelenksprothese;

Fig. 2 ist - teilweise im Schnitt - eine Ansicht
von Fig. 1 von links;

Fig. 3a und 3b gibt in stark vergrössertem Massstab ein Detail aus Fig. 2 im Schnitt wieder.

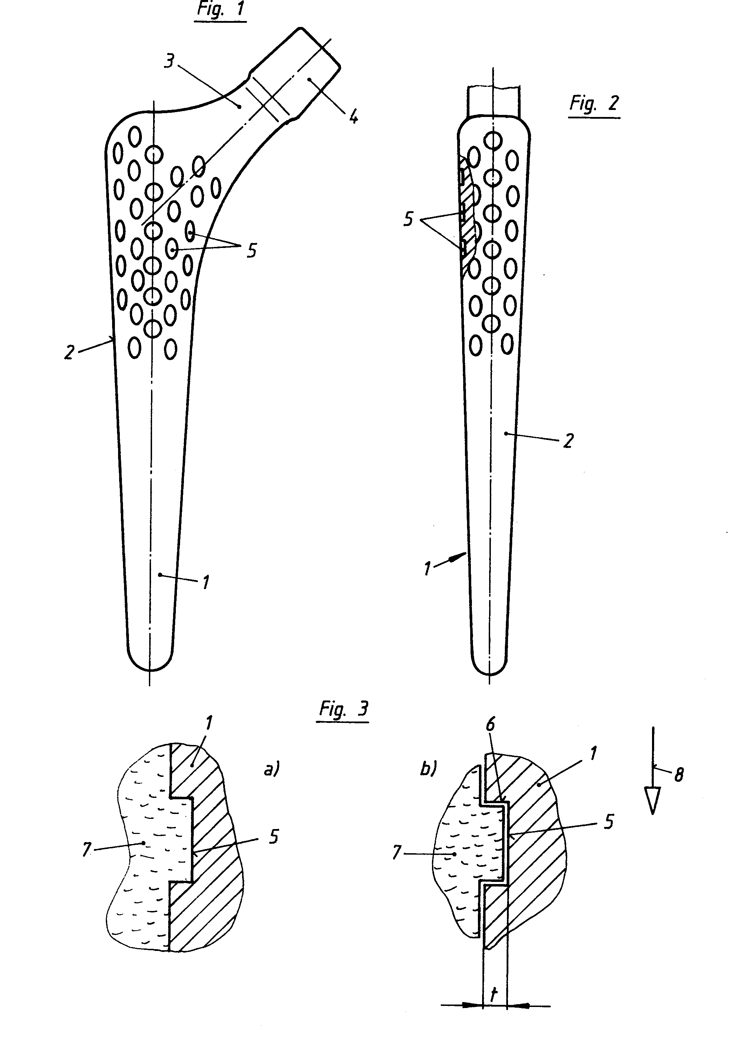

Der gerade Schaft 1 (Fig. 1), der aus Metall, vorzugsweise
aus Titan oder einer Titanlegierung, besteht, ist allseitig
abgerundet und erweitert sich konisch von seinem distalen
Ende nach proximal. Sein Querschnitt ist oval oder ellipsenähnlich und vergrössert sich von distal nach proximal kontinuierlich. Die laterale Schmalseite 2 führt in einem Bogen
zu einer horizontalen Schulter am proximalen Schaftende, die
ihrerseits in den Prothesenhals 3 übergeht. Dieser trägt
einen konischen Zapfen 4 für die Aufnahme des nicht dargestellten Gelenkkopfes.

Im proximalen Bereich sind in die "breiten" Seiten und in
die laterale Schmalseite 2 des Schaftes 1 Vertiefungen 5
eingearbeitet, die regel- oder unregelmässig auf der Oberfläche der genannten Seiten verteilt sein können. Der Querschnitt der Vertiefungen 5 ist der einfachen Herstellung
wegen kreiszylindrisch; erfindungsgemäss verlaufen die
Mantelflächen 6 (Fig. 3b) der Vertiefungen 5 senkrecht zur
Oberfläche des Schaftes 1. Ihre Tiefe beträgt beispielsweise
t = 1,5 mm (Fig. 3b).

Bei der Implantation füllt der noch nicht ausgehärtete Knochenzement 7
(Fig. 3a) die Vertiefungen 5 vollständig aus. Er schwindet
jedoch beim Aushärten, so dass - wie in Fig. 3b stark übertrieben dargestellt - ein "Spalt" den Knochenzement 7 von der Schaftoberfläche trennt. Aufgrund der senkrecht zur Schaftoberfläche verlaufenden Mantelflächen 6 der Vertiefungen 5 bleibt jedoch eine stufenartige Auflage erhalten, auf der sich der Knochenzement 7 in der Vertiefung 5 abstützen kann. Diese Auflage verläuft senkrecht zu der durch den Pfeil 8 angedeuteten Hauptbelastungsrichtung des Geradschaftes 1, so dass sich keine Kraftkomponente einstellt, die den Knochenzement 7 aus der Vertiefung 5 hinausdrängt.

Patentansprüche

1. Gerader, allseitiger abgerundeter Schaft zur Verankerung einer Gelenkendoprothese mit Hilfe von Knochenzement, welcher Schaft auf der Oberfläche mit einer Verankerungsstruktur aus einzelnen Vertiefungen versehen ist, dadurch gekennzeichnet, dass die Vertiefungen (5), die mindestens im proximalen Bereich des Schaftes (1) angeordnet sind, mit ihren Mantelflächen (6) mindestens nahezu senkrecht zur Oberfläche des Schaftes (1) verlaufen.

2. Schaft nach Anspruch 1, dadurch gekennzeichnet, dass die Tiefe (t) der Vertiefungen (5) absolut mindestens 1 mm beträgt.

3. Schaft nach Anspruch 1 oder 2, dadurch gekennzeichnet, dass der Durchmesser der Vertiefungen (5) das 2- bis 4-fache ihrer Tiefe (t) beträgt.

1/1

*Fig. 1*

*Fig. 2*

*Fig. 3*

a)

b)

0212084

0212084

Europäisches
Patentamt

EUROPÄISCHER RECHERCHENBERICHT

Nummer der Anmeldung

EP 86 10 7054

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | AU-B- 480 221 (TELECTRONICS) <br> * Abbildungen; Anspruch 1 * | 1 | A 61 F 2/30 |
| X | EP-A-0 106 945 (SULZER) <br> * Zusammenfassung; Abbildungen * | 1 | |
| A | DE-A-3 322 803 (ORTHOPLANT) <br> * Seite 8, Zeile 22 - Seite 9, Zeile 11; Abbildungen * | 1-3 | |
| E | EP-A-0 158 534 (FINSBURY) <br> * Abbildung; Seite 5, Zeilen 13-16 * | 1 | |
| A | US-A-3 740 769 (HABOUSH) | | |

RECHERCHIERTE SACHGEBIETE (Int. Cl.4)

A 61 F

Der vorliegende Recherchenbericht wurde für alle Patentansprüche erstellt.

| Recherchenort | Abschlußdatum der Recherche | Prüfer |
|---|---|---|
| DEN HAAG | 23-10-1986 | STEENBAKKER J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82